# EUROPÄISCHE PATENTSCHRIFT

(11) **EP 1 253 966 B1**
(45) Veröffentlichungstag und Bekanntmachung des Hinweises auf die Patenterteilung: **27.04.2011**
(21) Anmeldenummer: 00953113.8
(22) Anmeldetag: 28.07.2000
(51) Int. Cl.: A61P 17/00, A61P 19/10, A61P 9/10, A61P 13/12, A61K 31/565

(54) **SUBSTANZEN UND MITTEL ZUR POSITIVEN BEEINFLUSSUNG VON KOLLAGEN**
SUBSTANCES AND AGENTS FOR POSITIVELY INFLUENCING COLLAGEN
SUBSTANCES ET MOYENS PERMETTANT D'INFLUENCER POSITIVEMENT LE COLLAGENE

(30) Priorität: 13.08.1999 DE 19938421
(43) Veröffentlichungstag der Anmeldung: 06.11.2002
(73) Patentinhaber: Curadis GmbH, 91052 Erlangen (DE)
(72) Erfinder: Wieland, Heinrich, 79221 St. Peter (DE); Schmidt, Alfred, 79183 Waldkirch (DE)
(74) Vertreter: Schrell, Andreas
(86) Internationale Anmeldenummer: PCT/EP2000/007315
(87) Internationale Veröffentlichungsnummer: WO 2001/012206

(56) Entgegenhaltungen:
- EP-A- 0 684 235
- EP-A- 0 747 042
- EP-A- 0 776 661
- EP-A- 1 120 108
- WO-A-91/00731
- WO-A-92/18132
- WO-A-96/08231
- WO-A-96/40048
- WO-A-97/36570
- WO-A-99/62459
- WO-A-99/62480
- DE-A- 3 338 212
- US-A- 5 280 035
- US-A- 5 494 899
- US-A- 5 906 987
- US-A- 5 972 921
- DATABASE CHEMAB [Online] CHEMICAL ABSTRACTS SERVICE, COLUMBUS, OHIO, US; MORITA, KYOKO ET AL: "Effect of soybean isoflavone on bone metabolism" retrieved from STN Database accession no. 133:16833 XP002161790 & DAIZU TANPAKUSHITSU KENKYU (1999), 2, 76-82,
- THESEN: "formestane, a new aromatase inhibitor" PHARM. ZTG., Bd. 141, Nr. 26, 1996, Seiten 32-40, XP000986872

## Beschreibung

Die vorliegende Erfindung betrifft den Einsatz von Substanzen und Mitteln, die die peripher-lokale, gewebe- bzw. organ-zellspezifische Bildung von Sexualhormonen günstig beeinflussen. Die Erfindung betrifft insbesondere den Einsatz solcher Substanzen und Mitteln zur positiven kosmetischen Beeinflussung des Kollagens. Hierfür kommen Applikationen für Kollagen-haltige Körperpartien in Betracht, wie Haut, Sehnen, Fascien, Bänder, Knorpel, Knochen, Zahnbein, Arterien und Venen, Harngefäße und andere Gefäßwände. Daraus folgen nicht erfindungsgemäß überaus nützliche Möglichkeiten für die Prophylaxe und Therapie verschiedener Erkrankungen. "Positive Beeinflussung" im Sinne der Erfindung bedeutet vor allem eine Stabilisierung, Vermehrung und/oder Restaurierung des Kollagens bzw. der Kollagenfasern.

Zum Hintergrund der Erfindung wird nachfolgend der Zusammenhang der peripher-lokalen, gewebe- bzw. organzellspezifischen Bildung von Sexualhormonen beschrieben.

Testosteron gilt als Inbegriff des männlichen Geschlechtshormons. Seine Wirkungen werden über den sogenannten Androgenrezeptor vermittelt. Wie alle Steroidhormone wirkt Testosteron zusammen mit seinem Rezeptor als Transkriptionsfaktor (Roy 1995), der die Transkription reguliert. Androgenrezeptoren verändern nach Bindung des Androgens ihre Struktur und bewegen sich in den Zellkern zu entsprechenden Genen, derer Expression durch Androgene beeinflusst wird. Testosteron selbst bindet sich nicht besonders gut an den Androgenrezeptor, sondern muss hierfür erst innerhalb der Zelle leicht chemisch modifiziert werden. Das Enzym 5-alpha-Reduktase entfernt die einzige Doppelbindung im Steroidhormonmolekül und es entsteht Dihydrotestosteron (DHT), das eine zehnfach höhere Affinität zum Androgenrezeptor besitzt als Testosteron (Grino,1990). Dihydrotestosteron kann auch aus 5 alpha-Dihydroandrostandion entstehen, das seinerseits mit Hilfe der 5 alpha-Reduktase aus Androstendion entstanden ist. Dieser Weg überwiegt z.B. in der Genitalhaut von Mann und Frau bei weitem (Stanczyk 1990).

Diese erst im Zielorgan, d.h. peripher stattfindende Umwandlung verstärkt also die Testosteronwirkung erheblich. In der Haut besteht sie zum Beispiel in der Förderung des Haarwuchses (außer auf der Kopfhaut) und in der Steigerung der Aktivität der Talgdrüsen. Nur Zellen, die sowohl die 5-alpha-Reduktase-Aktivität als auch Androgenrezeptoren besitzen, lassen sich durch Testosteron in physiologischen Mengen entsprechend stimulieren. Das Testosteron stammt überwiegend aus den Hoden und zu einem kleinen Teil auch aus der Nebennierenrinde. Der Testosteronspiegel beträgt beim Mann 280 bis 1100 Nanogramm/ Milliliter, bei der Frau 15 bis 70 Nanogramm/ Milliliter (Östradiol: bis zu 0,45 Nanogramm/Milliliter).

Nicht nur für das Dihydrotestosteron, sondern auch für das Testosteron selbst existiert ein intrazellulärer peripherer Syntheseweg (Labrie 1995). Das dort gebildete Testosteron gelangt aber kaum in den Blutstrom, sondern übt seine Wirkung innerhalb der Zelle, in der es entstanden ist nach Umwandlung in DHT in derselben Zelle aus. Der Vorläufer des peripher gebildeten Testosterons wird von der Nebennierenrinde an das Blut abgegeben. Hierbei handelt es sich um das Dehydroepiandrosteron (DHEA), das im Blut in mikromolaren Konzentrationen vorhanden ist. Dehydroepiandrosteron wird innerhalb der Zelle, in die es auf einfache Weise durch simple Diffusion durch die Zellmembran hindurch eingedrungen ist, in ein anderes Molekül umgewandelt. Dabei wird die 3-beta Hydroxylgruppe in eine Ketogruppe umgewandelt und die Doppelbindung des Rings B wird in den Ring A transferiert (3-Betahydroxysteroid-Dehydrogenase/Isomerase, 3-beta HSD). Dabei entsteht Androstendion, von dem es zum Testosteron nur ein kleiner Schritt ist (Umwandlung der Ketogruppe an C-17 in eine Hydroxylgruppe mit der 17-Betahydroxysteroid-Dehydrogenase). Dieses Testosteron wird dann innerhalb derselben oder anderer Zellen in das potente Androgen DHT umgewandelt. Zellen, die die obengenannten zwei Enzyme besitzen, können sich also ihr Testosteron aus einem in relativ hoher Konzentration im Blut vorkommenden Vorläufer, dem Dehydroepiandrosteron selbst herstellen, es selbst weiter verarbeiten oder an die Umgebung abgeben, wo es auf Zellen treffen kann, die 5-alpha-Reduktase besitzen und damit DHT bilden können.

Das Ausmaß der Umwandlung von DHEA in Androstendion in peripheren Geweben hängt zum einen vom DHEA-Spiegel, zum anderen aber auch von der Aktivität der 3-beta HSD ab. Letztere wird sehr wahrscheinlich nicht nur im Hoden, sondern auch in peripheren Geweben, die die entsprechenden Rezeptoren besitzen, vom luteotropen Hormon (LH) stimuliert (Venencie 1999).

Bestimmte Strukturen im Gehirn können die Testosteronkonzentration fühlen. Wenn sie diese als zu gering empfinden, ergeht an die Hypophyse der Befehl, vermehrt LH auszuschütten. Dadurch wird dann auch die Androstendion- bzw. Testosteronbildung in entsprechenden peripheren Geweben wie z.B. der Haut stimuliert. Bei Vorhandensein von 5-alpha-Reduktase stellen sich dann vermehrt die Androgeneffekte ein.

Bei Frauen wird vom Gehirn statt der Konzentration der Androgene die der Östrogene gemessen. Eine Verminderung derselben führt in den entsprechend ausgestatteten Geweben ebenfalls über LH-Ausschüttung zu einer Steigerung der peripheren Umwandlung von DHEA in Androstendion. Außer in Testosteron kann Androstendion auch in Östron umgewandelt werden. Das hierfür verantwortliche Enzym ist die Aromatase, die nicht ganz so weit verbreitet ist wie das testosteronbildende Enzym, die 17-beta-HSD. Sie kommt ebenfalls in der Haut vor (Thiboutot, 1998, Theintz, 1989, Milewich 1988, Svenstrup 1990, Milevich 1990, Dijkstra 1987), aber auch in anderen gewebe- bzw. organspezifischen Zellen. Östron ist ein nur schwach wirksames Östrogen und muss zur vollen Entfaltung seiner Wirkung so wie das Testosteron erst in der Zielzelle zum wirksamen Hormon umgewandelt werden. Dies erledigt die17-beta-HSD, die Östron in das wesentlich wirksamere Östradiol umwandelt. Der Wirkmechanismus des Östradiols auf zellulärer Ebene entspricht dem des DHT. Es wirkt über intrazelluläre Hormonrezeptoren, die als Transkriptionsfaktoren selektiv die entsprechenden Gene aktivieren. Östradiol kann aber auch intrazellulär aus Androstendion nicht nur über Östron, sondern auch über Testosteron entstehen, das ebenfalls von der Aromatase in ein östrogen umgewandelt werden kann. Diesmal direkt in das wirksame Östrogen, das Östradiol. Testosteron kann also innerhalb bestimmter Zielzellen mit Hilfe jeweils eines einzigen enzymatisch katalysierten Schrittes in ein hochwirksames Androgen oder ein hochwirksames Östrogen umgewandelt werden, je nachdem welches Enzym aktiver ist. Um also in peripheren Geweben über Hormonwirkungen von Östrogenen oder Androgenen zu verfügen, benötigt der Organismus weder Eierstöcke noch Hoden, sondern nur eine Nebennierenrinde (Labrie 1995, Labrie 1997). Daher können sowohl Männer als auch Frauen in entsprechenden Geweben sowohl Östradiol als auch Testosteron herstellen. Da die Bildung der hochwirksamen Hormone und ihre Wirkungen in derselben Zelle stattfinden können, spricht man von "Intrakrinologie" (Labrie 1991).

Da die Aromatase weniger ubiquitär verteilt ist als die 17-betaHSD, kann es vorkommen, dass die zu aromatisierenden Steroide innerhalb anderer Zellen hergestellt wurden als Östron oder Östradiol. So sind im Eierstock die Thekazellen auf die Herstellung von androgenen Östrogenvorläufern spezialisiert und Östradiol wird aus diesen in den benachbarten Granulosazellen hergestellt (Tamaoka 1987, Roberts 1990). Dies ist durchaus sinnvoll, weil dann nur die Zellen, deren Zunahme mit dem Follikelwachstum verknüpft ist, Östradiol in das Blut sezernieren, dessen Konzentration dem Gehirn die Größe des Follikels mitteilt. Ab einer bestimmten Östradiolkonzentration im Blut kommt es dann zum Eisprung. Dieser wird durch einen plötzlichen Anstieg der FSH Konzentration im Blut ausgelöst, der durch das Überschreiten einer bestimmten Grenzkonzentration des Östradiols im Blut bewirkt wird. Die direkte Herstellung von Östrogenen aus DHEA findet in hohem Maße während der Schwangerschaft in der Plazenta statt. Der wesentliche Vorläufer ist die sulfatierte Form, das DHEA-S. DHEA-S stammt nicht aus der Nebennierenrinde der Schwangeren, sondern aus der des Feten (Gips 1980). Wenn die Anlieferung bzw. die Umwandlung nicht funktionieren, kommt es zu einer diagnostisch aussagekräftigen Verminderung bestimmter Abbauprodukte der Östrogene (17 Ketosteroide) im Urin. Wenn die DHEA freisetzende Sulfatase fehlt (angeborene Ichthyose, kommt nur bei Jungen vor), kommt es im Blut der Mutter nicht zu einem Anstieg der Östrogenkonzentration. Die extragonadale Sexualhormonbildung in der Haut spielt anscheinend schon beim Feten eine Rolle. Eine weitere Bedingung, bei der es trotz kaum vorhandener Östrogene im mütterlichen Blut dennoch zu einer normalen Spontangeburt kommt, ist der angeborene Mangel an Aromatase. Die Plazenta wandelt die Androgene nicht mehr in Östrogene um, und es wächst der Mutter sogar ein Schnurrbart. Dies mag auf der Stimulierung der extragonadalen Sexualhormonbildung in der Haut der Mutter beruhen, die vom humanen Choriongonadotropin (hCG) ausgeht. Diese gesteigerte Bildung ist wohl dafür verantwortlich, dass an den Stellen, an denen zu einer normalen Geburt Östradiol benötigt wird, diese aus den Vorläufern auch gebildet wird. Die vorwiegend von der Plazenta gebildeten Östrogene sind biologisch recht unwirksam. Ihre gesteigerte Bildung beruht wohl sowohl auf der hohen Konzentration der fetalen Vorläufer im Nabelschnurblut als auch auf der Stimulierung der Aromatase durch das Schwangerschaftshormon hCG, dessen beta-Kette mit der des FSH nahezu identisch ist und daher auch identische biologische Wirkungen ausübt. Also wird bei der schwangeren Frau die periphere Bildung von Sexualsteroiden in den Organen gesteigert, die über LH/hCG-Rezeptoren (oder möglicherweise über Rezeptoren für einen anderen Botenstoff) verfügen. Hierzu gehört sicher die Haut (Venencie 1999, You 2000).

Aufgabe der Erfindung ist es, die extragonadale Sexualhormonbildung günstig zu beeinflussen, um über die peripher-lokale, gewebe- bzw. organ-zellspezifische Präsenz oder Abwesenheit der Sexualhormone therapeutische Effekte zu ermöglichen.

Die Erfindung betrifft eine kosmetische Verwendung gemäß der Ansprüche.

Im Rahmen der Erfindung wurde überraschender Weise gefunden, dass das Kollagen in den betreffenden, Kollagen-haltigen Körperteilen positiv beeinflusst werden kann, indem eine Substanz eingesetzt wird, die die Fähigkeit zur Hemmung der Bildung und/oder der Wirkung von Östrogenen besitzt. Nach Applikation der Substanz oder eine diese Substanz enthaltende Zusammensetzung wird das Kollagen in den Kollagen-haltigen Körperteilen stabilisiert, vermehrt und/oder restauriert.

Mit diesem Ansatz geht die vorliegende Erfindung einen völlig neuen Weg. Zentraler Punkt dieses Ansatzes ist ein gezielter Eingriff in die peripher-lokale, gewebe- bzw. organ-zellspezifische Bildung von Sexualhormonen mittels hierfür besonders geeigneter Substanzen, und zwar in erster Linie solchen, die die Bildung und/oder der Wirkung von Östrogenen hemmen. Da die Aromatase als ein Schlüsselenzym in diesem Kontext festgestellt wurde, dienen erfindungegemäß Aromatase-Inhibitoren, die auch eine 5- alpha-Reduktase-Inhibitorwirkung besitzen, als Substanzen zum Einsatz in der vorliegenden Erfindung gemäß der Patenansprüch. Ebenfalls günstig, vor allem bei Anwendung in der Haut, kann sich die gleichzeitige oder ergänzende Hemmung der Bildung von Dihydrotestosteron auswirken. Hierfür kommen vorzugsweise 5-alpha-Reduktase-Inhibitoren, aber auch alpha-Rezeptorblocker in Frage.

Es wurde überraschend festgestellt, dass nach Einwirkung der genannten Substanz(en) bzw. der diese Substanz(en) enthaltenden Zusammensetzung ein positiver Einfluss auf das Kollagen, insbesondere den Gehalt an Kollagenfasern, in der Kollagen haltigen Körperregion wie der Haut ausgeübt wird, so dass diese Körperregion fester wird. Anhand von Biopsien wurde festgestellt, dass der Anteil an Kollagenfasern zunahm. Es wird angenommen, dass - im fundamentalen Kontrast zum natürlichen Einfluss über den Östrogen-Spiegel im Blut sowie im Gegensatz zur bekannten Östrogenersatztherapie (HRT) - ein günstiger Einfluss auf das Kollagen an Ort und Stelle erzielt werden kann, wenn durch die erfindungsgemäße Applikation die lokale extragonadale Östrogenbildung und/oder die lokale Wirkung der Östrogene durch den Einsatz der speziellen Substanz bzw. der diese Substanz enthaltenen Zusammensetzung gehemmt wird.

In einem weiteren Aspekt der Erfindung wurde festgestellt, dass negative Einflussfaktoren, die sich auf den Kollagengehalt und -stabilität nachteilig auswirken können, durch die Verwendung der Substanz bzw. der diese Substanz enthaltenden Zusammensetzung zumindest teilweise kompensiert und somit schädigende Auswirkungen auf den menschlichen Körper abgemildert werden können. Als solche negativen Einflussgrößen wurden insbesondere die gesteigerte Ausschüttung von LH, die Bildung von Vitamin D infolge Sonneneinstrahlung sowie die übermäßige Präsenz oder die Gabe von Glukokortikoiden identifiziert.

Aus dem vorstehend genannten Konzept ergeben sich bedeutende Implikationen und nützliche kosmetische und medizinische Anwendungen, die unten beispielhaft näher erläutert werden. Für die nicht erfindungsgemäße medizinischen Anwendungen können die geeigneten Substanzen zur Herstellung eines Mittels oder pharmazeutischen Formulierung zusammen mit für die jeweilige Applikationsart üblichen pharmazeutisch akzeptablen Zusatzstoffen eingesetzt werden und in den therapeutischen Behandlungen angewandt werden.

Unter dem Begriff "Östrogene" sind alle natürlichen, weiblichen Sexualhormone mit östrogener Wirkung zu verstehen, wie Östradiol, Östron und Östrol.

Als im Hinblick auf Östrogene bildungs- und/oder wirkungshemmende Substanzen kommen insbesondere zwei Klassen von Substanzen in Betracht, die im Folgenden näher beschrieben werden.

Einmal sind dies Anti-Östrogene, d.h. solche Substanzen, die Östrogenrezeptoren blockieren und somit als Antagonisten die Wirkung von Östrogen hemmen.

Ferner sind dies Substanzen, die lokal die extragonadale Östrogen-Bildung hemmen können. Hierfür kommen vor allem steroidale und nicht-steroidale Inhibitoren der (Cytochrom-p450)-Aromatase in Frage. Die Aromatase stellt das zentrale Enzym dar, welches die chemische Umwandlung der aus der Nebenniere stammenden und über das Blut transportierten Vorläufer-Moleküle (wie Dehydroepiandrosteron (DHEA) und Androstendion) in Östrogene katalysiert. Die Hemmung dieses Enzyms führt folglich zu einer lokalen in *situ*-Hemmung der Östrogen-Bildung. Aufgrund ihrer besonders vorteilhaften Wirkungsweise sind die in den Ansprüchen genannten Aromatase-Inhibitoren zum Einsatz bei der erfindungsgemäßen Verwendung vorgesehen.

Beispiele für Aromatase-Inhibitoren schließen folgende Substanzen ein:
Steroidale Aromatasehemmer:
   4-Hydroxyandrost-4-en-3,17-dion (Formestan und Lentaron),
   6-Methylenandrostra-1,4-dien-3,17-dion (Exemestan),
   10-(2-Propynyl)estr-4-en-3,17-dion (MDL 18962)
   7 alpha substituierte Androstendion-Derivate
   1,4,6-androstatriene-3,17-dion (ATD)
   10-Oxiran- und 10-Thiirane substituierte Androgene
   10-Propargylestr-4-ene-3,17-dione
   10-propargylestr-4-ene-3,17-propionate 10-(2-propynyl)-Derivat
   13-retro-Antiprogestine
   14 alpha-hydroxy-4-androstene-3,6,17-trione (14 alpha-OHAT)
   16- oder 19-substituierte Androst-4-ene
   19-(Cyclopropylamino)-androst-4-en-3,17-dion
   19-(Ethyldithio)-androst-4-ene-3,17-dione (ORG 30958)
   19-Oxiranyl- and 19-Thiiranyl-Steroide
   19-Thiomethyl- and 19-Azido-androstenedion
   1-Methyl-androsta-1,4-diene-3,17-dione (Atamestan)
   2,2-Dimethyl-4-hydroxy-4-androstene-3,17-dion
   3 alpha-methoxyandrost-4-ene-6,17-dione
   3 beta-hydroxyandrost-4-en-6-one-Derivate
   3-Deoxyandrogen-19-Oxygenierderivate von 3-oxo-17 betacarboxamido-Steroide
   4-(Phenylthio)-4-androstene-3,17-dion
   4-(Thio-substituiertes)-4-androstene-3,17-dion
   4-Acetoxy-4-androsten-3,17-dion
   4-Aminoandrostenedion
   4-Androstene-3,6,17-trion
   4-Hydroxyandrostenedion (4-OHA, CGP 32349)
   4-Methoxy-4-androstene-3,17-dion
   4-Oxygenierte Androst-5-en-17-one und deren 7-oxo-Derivate
   4-Thiosubstituierte Derivate von 4-Androsten-3,17-dion
   4-Thiosubstituierte-4-androsten-3,17-dion-Derivate
   5 alpha-Dihydronorethindron (ein Metabolit von Norethindron)
   5 alpha-reduzierte C19-Steroide
   5 alpha-Androstan-17-ones mit oder ohne eine Carbonylfunktion an C-3 und/oder C-6
   6 alpha,7 alpha-Cyclopropanderivate von Androst-4-en
   6 alpha-Fluorotestosteron
   6 beta-Propynyl-substituierte Steroide
   6,7-Aziridinylsteroid und verwandte Verbindungen
   6-Alkylanaloge von delta 1,4,6-Androgenen
   6-Alkylanaloge of delta 4,6-Androgenen
   6-Alkyl- und 6-Arylandrost-4-en-3,17-dione
   6-Alkylandrost-4-en-3,17-dione von 7 alpha- and 7
   beta-arylaliphatisch-substituierten Androst-4-en-3,17-dionen
   6-Alkylandrosta-4,6-dien-3,17-dione und deren 1,4,6-trien-Analoga
   6-Alkyl-substituierte Androgene
   6-Phenylaliphatisch-substituierte C19-Steroide mit 1,4-dien-,
   4,6-dien- oder 1,4,6-trien-Struktur
   6-Bromoandrostenedion
   6-Hydroximinoandrostenedion
   6-Methylenandrosta-1,4-dien-3,17-dion (FCE 24304)
   6-Methylenandrosta-1,4-dien-3,17-dion (FCE 24304)
   6-Phenylaliphatisch-substituierte Androst-4-en-3,17-dione
   6-substituierte Androst-4-en-Analoga
   7 alpha-(4'-Amino)phenylthio-4-androsten-3,17-dion
   7 alpha-substituierte Androsta-1,4-dien-3,17-dione
   7 alpha-substituierte Androstenedione
   7 alpha-(4'-Amino)phenylthio-4-androsten-3,17-dion
   7 alpha-arylaliphatische Androsta-1,4-dien-3,17-dione
   7 alpha-substituierte Androstenedione
   7 substituierte 4,6-Androstadien-3,17-dione
   7 substituierte Steroide
   Androst-4-en-3,6-dionderivative
   Androst-5-ene-7,17-dion 19-nor- und 5 beta,6 beta-epoxy-Derivative
   A-or B-ring-substituierte Derivative von Androst-4-en-3,6,17-trion
   A-ring verbrückte Steroid
   Bromoacetoxy 4-androsten-3-one
   delta 1,4,6-Androgene
   delta 4,6-Androgene
   epimere 6-Hydroperoxyandrostendione
   estr-4-ene-3, 17-dion (MDL 18 962),
   estr-4-ene-3,6,17-trione
   Flavonoide
   RU486

Nicht-steroidale Aromatasehemmmer:
6-[(4-Chlorophenyl)(1H-1,2,4-triazol-1-yl)-methyl] 1-methyl-1H-benzotriazol (Vorazol),
2,2'-[5-(1H-1,2,4-triazol-1-yl methyl)-1,3-phenylen]bis(2-methylproprionitril) (Arimidex),
4-[1-(Cyanophenyl)-1-(1,2,4-triazolyl)methyl]benzonitril (Letrozol),
(4-(5,6,7,8-Tetrahydro-imidazo-[1,5a]-pyridin-5-yl) benzonitril Monohydrochlorid (Fadrozol) Pyridoglutethimid (Rogletimid). Aminogluthetimid
1,2-Imidazolylmethylcyclopentanol-Derivative
1-[(Benzofuran-2-yl)phenylmethyl]-triazole and -tetrazole
1-[Benzofuran-2-yl)-phenylmethyl]-imidazole (substituiert)
1-(Benzofuran-2-ylmethyl)imidazole von N,N-disubstituierten-5-aminopyrimidin-Derivaten
1-Imidazolyl(alkyl)-substituierte Di- and Tetrahydroquinoline
1-Pentyl-3-(4-aminophenyl)pyrrolidin-2,5-dion
1-Phenyl-3-azabicyclo[3.1.0]hexan-2,9-dion
1-Phenyl-3-azabicyclo[3.1.0]hexan-2,4-dion und Analoge
3-alkylierte 3-(4-Aminophenyl)piperidin-2,6-dione
3-cycloalkyl-substituierte 3-(4-Aminophenyl)piperidin-2,6-dione
3-Ethyl-3-(4-pyridyl)piperidin-2,6- und 5-Alkylderivative
3-Ethyl-3-(4-pyridyl)piperidin-2,6-dion-Analoge
4-Amino-4H-1,2,4-triazol-Derivative
4-Cyclohexylanilin Aminoglutethimid
Benzimidazol- und Imidazol-Verbindungen
delta 1,4-Bisnorcholadiensäure
delta 1-Testolacton
Imidazolderivative von pyrrolidonischen and piperidonischen Imidazolyl-1,3,5-triazinen
MR 20492 and MR 20494 (zwei Indolizinonderivative)
Pyridyl-substituierte Indanone, Indane und Tetraline
s-Triazinderivat SEF19
Substituierte Pyridine
Testololacton

Andere Aromatasehemmer:
8-Bromo-cyclisches Adenosinemonophosphat FR901537
Hexamethylmelamin-Derivat (SAE9)
Insulin sensitizer Troglitazone und Ketoconazole Letrozole (CGS 20267)
Mefloquin
MPV-2213ad
N-n-Octanoylnornicotin und andere Nornicotinderivate
Org 33201
R 76713 und R 76713
Sesquiterpenlactone
SH 489
TAN-931
Thyroidhormone
Tobakalkaloidderivative
YM511

Hinsichtlich der Bezeichnungen dieser Substanzen sowie deren Verfügbarkeit siehe beispielsweise "Rote Liste", Editio Cantor, Aulendorf (DE), (1999).

Solche Aromatase-Inhibitoren sind an sich bekannt, hauptsächlich als systemisch eingesetzte Wirkstoffe zur medizinisch-therapeutischen Behandlung von Brustkrebs. In diesem Zusammenhang wird verwiesen auf die Übersichtsartikel von A.M.H. Brodi in: "J. Steorid Biochem. Molec. Biol.", Vol. 49, No. 4-6, pp. 281-287 (1994), sowie P. E. Goss und K.M.E.H. Gwyn in: "Journal of Clinical Oncology", Vol. 12, No. 11, pp. 2460-2470 (1994). Zur Bestimmung der Aromatase-Inhibition und der nachfolgenden Östrogenreduzierung wird auf die in den genannten Übersichtsartikeln angegebenen, weiteren Literaturnachweise verwiesen, s. beispielsweise A.M.H. Brodi et al. in: "J. Steroid Biochem. Molec. Biol.", Vol. 7, pp. 787-793 (1976), und D.A. Marsh et al. in: "J. Med. Chem.", Vol. 28, pp. 788-795 (1985).

Spezielle Azolderivate und deren aromatasehemmende und antimycotische Wirkung werden ferner beschrieben in der EP-A-0 575 210.

Es hat sich gezeigt, dass in Soja-Gycinen (INCI-Name nach dem Linné-System) Substanzen mit aromatasehemmenden Eigenschaften enthalten sind, und dass diese aus Soja-Glycinen stammenden Aromatase-Inhibitoren eingesetzt werden können. Diese aus Soja-Glycinen stammenden Aromatase-Inhibitoren können leicht erhalten werden durch Bereitstellung von "Glycine Soja" (Sojabohnenöl bzw. -extrakt oder Sojasterol) und anschließender Isolierung der Komponente mit aromatasehemmender Wirkung über gängige Trennmethoden, wie der Flüssigkeitschromatographie, insbesondere mittels der HPLC.

Es hat sich ferner herausgestellt, dass sich die Aromatase-Inhibitorwirkung der Soja-Glycine steigern lässt, wenn die Soja-Glycine oxidativ behandelt werden.

Die Darstellung dieser aus Soja-Glycinen stammenden, oxidierten Form erfolgt auf einfache Weise durch Oxidation der Soja-Glycine (Sojabohnenöl bzw. -extrakt oder Sojasterol) und anschließender Isolierung der Komponente mit aromatasehemmender Wirkung über gängige Trennmethoden, wie der Flüssigkeitschromatographie, insbesondere mittels der HPLC.

Die Oxidation kann auf enzymatische Weise, zum Beispiel gemäß der von Y.Fujimoto et al. in: "J. Am. Chem. Soc.", Vol. 104, pp. 4718-4720 (1982) beschriebenen Methode, oder auf chemischem Wege, wie zum Beispiel gemäß der von P.Welzel in: "Tetrahedron", Vol. 41, No. 20, pp. 4509-4517 (1985), beschriebenen Methode.

Als Beispiele für Substanzen der Anti-Östrogenklasse sind insbesondere die nicht-steroidalen Östrogen-Antagonisten Tamoxifen (Z-2-[4-(1,2-Diphenyl-1-butenyl)-phenoxy]-N,N-dimethylamin) und Aminoglutethimid (3-(4-Aminophenyl)-3-ethyl-2,6-piperidin-dion) sowie deren Analoga und Derivate, beispielsweise das 3-Hydroxytamoxifen, das 4-Hydroxytamoxifen sowie das 7 α-Alkyl-Sulfinyl-Tamoxifen-Analoge (ICI 182,780), zu erwähnen.

Hinsichtlich der Bezeichnungen dieser Substanzen, deren Verfügbarkeit sowie weiterer geeigneter Anti-Östrogene, siehe beispielsweise "Rote Liste", Editio Cantor, Aulendorf (DE), (1999).

Auch diese Anti-Östrogene sind bisher vornehmlich im Zusammenhang mit der systemisch therapeutischen Behandlung von Brustkrebs beschrieben worden.

Um den Eingriff in die extragonadale, zelluläre Sexualhormonbildung oder -wirkung noch gezielter zu ermöglichen und besser abstimmen zu können, kann eine oder mehrere der oben beschriebenen Substanzen zur Hemmung der Bildung und/oder Wirkung von Östrogenen mit einem weiteren Wirkprinzip derart kombiniert werden, dass zusätzlich oder gleichzeitig die Bildung und/oder die Wirkung von Dihydrotestosteron gehemmt wird. Dies geschieht durch den Einsatz eines 5-alpha-Reduktase-Inhibitors oder eines alpha-Rezeptorblockers, wobei der Einsatz von 5-alpha-Reduktase-Inhibitoren stark bevorzugt ist.

Beispiele für 5-alpha-Reduktase-Inhibitoren, je nach Typ unterschieden, schließen ein:
Typ 1-Hemmer:
   LY191704 (Benzochinolinon)
   4,7 beta-Dimethyl-4-azacholestan-3-on (MK-386) und verwandte 4-Azasteroide
   Benzo[c]chinolizin-3-on
Typ 2-Hemmer:
   Benzophenon- and Indolcarboxylsäuren
   N-tert-Butyl-3-oxo-4-aza-5α-androst-1-en-17-β-carboxamid (Finasterid)
Duale Hemmer (Typ 1 und Typ 2):
   3-Carboxy-20-keto-Steroide
   6-Azasteroid
   4-Aza-3-oxo-5 alpha-androst-1-en-17 beta-N-aryl-6-Azasteroide FK143
Nichtsteroidale Hemmer:
   4-(1-Benzoylindol-3-yl)buttersäuren
   4-[3-[3-[Bis(4-isobutylphenyl)methylamino]benzoyl]-1H-indol-1-yl]-buttersäure
   Benzanilid-Derivate
   Carbamoylalkenyl)phenyloxy carboxylsäure-Derivate Ethyl-4-(1-methyl-2-oxopiperid-5-yl)benzoat
   FK143
   N,N-bis(1-Methylethyl)-4-[3-(1,2-dihydro-1-methyl-2-oxopyrid-5-yl)propyl]benzamid
   Phenoxybenzoesäure-Derivate
   Carboxamid- und Phenylalkyl-substituierte Pyridone und Piperidone
   Natrium-4-[2-(2,3-dimethyl-4-[1-(4-isobutylphenylethoxy] benzolamino)phenoxy]butyrat (ONO-3805)
   (Z)-4-2-[[3-[1-(4,4'-Difluorobenzhydryl)indol-5-yl]-2-pentenoyl]-amino]phenoxy]butyric acid (KF20405)
Steroidale Hemmer:
   17 beta-(N,N-Diisopropylcarbamoyl)estra-1,3,5(10)-trien-3-sulfonsäure
   17 beta-Carbamoyl-1,3,5(10)-estratrien-3-carboxylsäuren 17 beta-N,N-Diethylcarbamoyl-4-methyl-4-aza-5 alpha-androstan-3-on (4-MA)
   17 beta-N-(2-Methyl-2-propyl)-carbamoyl-androst-3,5-dien-3-carboxylsäure
   3-ndrosten-3-carboxylsäuren (steroidale Acrylate), 3-Carboxy-17 beta-substituierte Steroid
   4-aza-3-oxo-Steroidfamilie
   4-Hydroxy-androstenedion
   4-Methyl-4-aza-5 alpha-pregnan-3-on-20(S)-carboxylat
   6-Methylen-progesteron-, -androsten- und -androstan-Derivate Finasterid
   Progesteron
   Natrium-4-methyl-3-oxo-4-aza-5 alpha-pregnan-20 (S)-carboxylat
   Steroidale A-Ring-Arylcarboxylsäuren
   TZP-4238 (steroidales Antiandrogen)

Als Beispiel für einen alpha-Rezeptorblocker ist R-(-)-5-{2-[2-(2-Ethoxyphenoxy)ethylamino]peropyl]-2-methoxy-benzolsulfonamid (Tamsulosin) zu erwähnen.

Auch diese Substanzen zur Hemmung der Bildung und/oder Wirkung von Dihydrotestosteron sind an sich bekannt, allerdings lediglich zur Behandlung benigner Prostata-Hyperplasie (s. "Rote Liste", Editio Cantor, Aulendorf (DE), (1999)).

Als besonders nützliche und daher erfindungsgemäße Substanzen kommen solche in Betracht, die sowohl eine Aromatse- als auch eine 5-alpha-Reduktase-Inhibitorwirkung entfalten können. Als Beispiel für Substanzen mit dieser bifunktionellen Fähigkeit ist das dem Androstendion sehr ähnliche Sterol 4-Hydroxyandrostendion und deren Derivate, z.B. das o.g. Formestan, zu nennen.

Da im Rahmen der Erfindung die gleichzeitige Hemmung sowohl der Östrogen- als auch der Dihyarotestosteron-Bildung bzw. -Wirkung als neu und signifikant in erster Linie, wie unten noch näher erläutert wird, für topische Anwendungen an der Haut und für Haarwachstumsregulierungen erkannt wurde, wird erfindungsgemäß zusätzlich eine topische, d.h. zum Auftragen auf die Haut bestimmte Zusammensetzung zur Verfügung gestellt, die eine oder mehrere Substanz(en) die in den Ansprüchen genannt sind derart umfasst, dass die Bildung und/oder die Wirkung von Östrogenen und gleichzeitig die Bildung und/oder der Wirkung von Dihydrotestosteron gehemmt ist. Eine solche Zusammensetzung ist vor allem in einer lokal topischen Formulierung kosmetisch einsetzbar. Dieses kombinierte Wirkprinzip kann durch eine Kombination von Substanzen erhalten werden, die jeweils die Fähigkeit der Hemmung der Östrogenbildung bzw. - Wirkung einerseits bzw. die Fähigkeit der Hemmung der Dihydrotestosteronbildung bzw. -wirkung andererseits besitzen, wobei die oben genannten Substanzen mit den jeweiligen Funktionen geeignet sind. Wegen einer verbesserten Steuerbarkeit ist in diesem Zusammenhang eine Kombination eines Aromatase-Inhibitors mit einem 5-alpha-Reduktase-Inhibitor möglich. Dabei ist eine Ausgestaltung erfindungsgemäß , wenn wie oben beschrieben eine eingesetzte Substanz bifunktionell ist und sowohl Aromatase-Inhibitorals auch 5-alpha-Reduktase-Inhibitor-Eigenschaften aufweist.

Die oben beschriebene Substanz bzw. die Substanzen Können nicht erfindungsgemäß in üblichen Arzneiformen verabreicht werden. Je nach Wunsch oder Wahl enthalten die Arzneiformen dementsprechend die für eine orale oder eine topische Applikation, für Injektionen, für Inhalierungen oder zur Transdermaltherapie üblichen und bekannten Zusatzstoffe. Bevorzugt ist die Arzneiform als zur topischen Applikation oder Transdermaltherapie, zur Injektion oder zur Inhalation geeignet ausgestaltet.

Die Menge der Wirksubstanz zur Hemmung der Östrogenbildung bzw. -wirkung in solchen Formulierungen sind nicht kritisch und können dem jeweiligen Anwendungsfall angepasst werden. Geeignet ist beispielsweise ein Wirkstoffgehalt in der gesamten Zusammensetzung von 0,0001 bis 10 Gewichtsprozent (Gew.%), vorzugsweise 0,001 bis 5 Gew.% und insbesondere 0,3 bis 2 Gew.%. Die weiteren, gegebenenfalls vorhandenen Zusatzstoffe können in den für die jeweiligen Formulierungen üblichen Mengen eingesetzt werden. Entsprechendes gilt für die wahlweise zusätzliche Verwendung der Wirksubstanz zur Hemmung der Dihydrotestosteronbildung bzw. -wirkung.

Wenn sich unterschiedliche Wirkmechanismen zur Steuerung der Östrogenbildung bzw. wirkung und ggf. der Dihydrotestosteronbildung bzw. -wirkung gegenseitig ergänzen und günstig beeinflussen sollen, werden die hierfür geeigneten, oben beschriebenen Substanzen nicht erfindungsgemäß in einem ausgewogenen Verhältnis zueinander verwendet, um einen gewünschten Effekt zu erzielen. Das bei der Kombination zu verwendende Mengenverhältnis kann dabei den jeweiligen Bedürfnissen angepasst werden. So kann zum Beispiel jeweils die eine Substanzart oder die andere Substanzart überwiegen, je nachdem, welcher Wirkungsweg vorrangig angestrebt wird. Das gewichtsmäßige Mengenverhältnis der einen zur anderen Substanzart liegt beispielsweise in einem Bereich von 90/10 bis 10/90, insbesondere in einem Bereich von 60/40 bis 40/60. Verschiedene Aspekte der vorliegenden Beschreibung beruhen auf bestimmten Zuständen im menschlichen Körper, die sich durch einzelne oder mehrere der folgenden Kriterien auszeichnen:
- gesteigerte Aktivität der Aromatase,
- erhöhte Expression oder Bildung von LH und/oder hCG im menschlichen Körper,
- erhöhte Expression oder Bildung von Glukokortikoiden im menschlichen Körper, oder die therapeutische Gabe von Glukokortikoiden, und
- erhöhte Bildung von Vitamin D, beispielsweise infolge Sonneneinstrahlung.

Diese Zustände können spontan oder permanent und ferner inhärent durch natürliche oder pathologische Umstände vorhanden sein oder durch äußere Einwirkungen auftreten. Als gemeinsame Basis dieser beeinflussenden Kriterien ist die potentielle Stimulierung der an der extragonadalen Sexualhormonbildung beteiligten Enzyme zu sehen, wozu die Aromatase und möglicherweise auch die 5-alpha-Reduktase gehören. Einer durch diese Stimulierung bedingte Abnahme oder Schwächung des Kollagens wird jedoch durch den erfindungsgemäßen Einsatz der genannten Substanzen entgegengewirkt. Zu den aromatasehaltigen Organen oder Geweben gehören Haut, Bindegewebe, Knochen, Gefäßwände (sogar die Vena Cava enthält in ihrer Wand Aromatase [Sasano 1999]), Blutzellen (vor allem Makrophagen), Muskeln, Uterus, Gehirn und andere mehr.

Unter Beachtung dieser genannten Kriterien ergeben sich nicht erfindungsgemäß somit Anwendungsprofile, die wegen der hohen Prävalenz der zugrundeliegenden Krankheiten große Bedeutung haben, etwa gegen Osteoporose, Krampfadern und Ulcus Cruris, Arteriosklerose, Herzinfarkt, Harninkontinenz. Besondere Bedeutung erhalten wegen ihres Kollagengehaltes in diesem Zusammenhang Haut, Sehnen, Knochen und die Wände von Gefäßen und der ableitenden Harnwege.

Auch können je nach Zustand bestimmte Personengruppen besonders betroffen sein, z.B. postmenopausale Frauen.

Die Ursachen hierfür sind letztlich nicht gesichert, jedoch kann folgendes vermutet werden: Die Aktivität der an der extragonadalen Sexualhormonbildung beteiligten Enzyme lässt sich durch LH oder hCG stimulieren. Hierzu gehört natürlich auch die der Aromatase. Die extragonadale Sexualhormonbildung lässt sich aber nicht nur durch Steigerung der daran beteiligten Enzyme via LH stimulieren, sondern auch über die Bereitstellung erhöhter Vorläufermengen. In der Nebennierenrinde befinden sich LH-Rezeptoren. Diese sitzen auf steroidogenen Zellen, die DHEA bilden (Pabon 1996). Die Aktivität der Aromatase lässt sich ferner spezifisch durch Glukokortikoide stimulieren (Harada 1992), die die Transkription der m-RNA der Aromatase steigern. Der Promoter des Exons 1, das gewebsspezifisch exprimiert wird, besitzt in Zellen der Haut, den Stromazellen des Fettgewebes und in Osteoblasten ein glukokortikoidresponsives Element, an das der Glukokortikoidrezeptor bindet und die Transkription des Aromatasegens steigert (Zhao 1995). Die Postmenopause ist ein Zustand mit permanent erhöhter Konzentration an LH und dessen Freisetzungshormon LHRH oder GnRH, das die Ausschüttung des LH aus der Hypophyse steuert. Andauernd erhöhte LH Konzentration sollte in den Geweben, die hierauf reagieren können und Aromatase besitzen, zu einer andauernd erhöhten lokalen Östradiolproduktion führen. Dies hätte für deren Gehalt an Kollagenfasern eine Verminderung zur Folge.

Im folgenden werden einzelne Beispiele für besonders geeignete Anwendungsfälle und Behandlungsmethoden beschrieben.

### Haut

Eine wesentliche Veränderung der Haut bei der Schwangerschaft sind die Schwangerschaftsstreifen, die als Dehnungsstreifen interpretiert werden. Ähnliche Streifen findet man aber auch beim Morbus Cushing, einer Bedingung mit permanent erhöhten Blutspiegeln an Glukokortikoiden, hervorgerufen durch Ersatz der zyklischen Schwankungen des Blut-ACTH Spiegels durch ein Plateau der ACTH Konzentration. Das Kennzeichen der Haut der Schwangerschaftsstreifen ist eine Verminderung des Kollagengehaltes. Die Gene für Typ I und Typ II Kollagen sind im Vergleich zur normalen Haut nur zu 10% exprimiert (Lee 1994). Aromatase kommt in der Haut sowohl in Fibroblasten als auch Keratinozyten vor (Berkowitz 1984, Fujimoto 1986, Bisat 1989, Harada 1992, Hughes 1997, Lachgar 1999). In beiden Zellen kann ihre Expression durch Glukokortikoide (Harada 1992 Berkowitz 1992, Ida 1991, Svenstrup 1990, Berkowitz 1981, Hughes 1997) gesteigert werden. So kommt eine Übersichtsarbeit aus dem Jahre 1984 zum Ergebnis, dass Glukokortikoide, aber auch Östradiol kollagenvermindernde Wirkungen haben (Borel 1984). In letzter Zeit konnte die kollagenvermindernde Wirkung des Östradiols auf molekularer Ebene direkt in den Mesangiumzellen der Nierenglomerula gezeigt werden (Neugarten 1999, Silbiger 1999, Kwan 1996).

Als gängige Lehrmeinung gilt allerdings, dass die Haut ein Östrogen-abhängiges Organ ist, wobei Östrogene nicht zu einer Verminderung, sondern zu einer Vermehrung der Kollagenfasern in der Haut führen sollen.

Das Kollagen der Haut ist im wesentlichen vom Typ I. So fanden M.L. Barklink et al. ("J. Appl. Physiol." 1993, 74(2), S. 727-732) heraus, dass die Knochenmasse und der Haut-Kollagengehalt mit zunehmendem Alter abnehmen. Es wurde festgestellt, dass ein Zusammenhang besteht zwischen dem verminderten Kollagengehalt der Haut und der Verminderung des Östrogen-Spiegels im Blut, die mit zunehmendem Alter in der Menopause einhergeht. D. Gruber et al. ("Klin. Wochenschrift" (Wien) 1995, 107, S. 622-625) berichten, dass die Östrogen-abhängige, postmenopausale Verminderung im Kollagengewebe mittels Ultraschall beurteilt werden kann und nehmen an, dass eine erfolgreiche Therapie durch Optimierung der Dosen einer Östrogenersatztherapie (hormone replacement therapy, HRT) möglich ist. Auch andere Experten (s. z.B. "Therapie" 1996, 51, S. 67-70; und "Dermatology" 1996, 193, S. 289-294) versuchen, der insbesondere postmenopausal auftretenden Alterung der Haut mit einer HRT zu begegnen.

Jedoch gab es keine Bestätigung dafür, dass Östrogene den Kollagengehalt der Haut vermehren können. So führen Östradiol-Implantate zu einer deutlichen Verminderung der unreifen "Crosslinks" Hydroxylysinorleuzin (Holland 1994). Der prozentuale Kollagengehalt und der Anteil an reifen "Crosslinks" (Histidinohydroxylysinorleuzin) der Haut verändert sich nicht.

Die HRT (hormone replacement therapy) verändert weder Menge noch Syntheserate des Kollagens der Haut (Haapasaari 1997). Vereinzelt wird gefunden, dass der relative Anteil des Kollagens vom Typ III nach Östrogengabe zunehmen soll (Savvas 1993, Schmidt 1996).

Bei der Behandlung von Kollagenmangelerscheinungen der Oberhaut, wie z.B. Erschlaffung der Oberhaut, Faltenbildung und Überdehungsstreifen, wurde erfindungsgemäß festgestellt, dass der positive Einfluss auf das Kollagen lokal in der Kutis ausgeübt wird, also in Epidermis und Korium. Es ergibt sich also der zu der offiziellen Lehrmeinung im Widerspruch stehende, aber durch experimentelle Untersuchungen gestützte Befund, dass Östradiol lokal direkt eine kollagenvermindernde Wirkung hat, wohingegen es erfindungsgemäß gelingt, durch lokale Hemmung der Aromataseaktivität in der Haut durch die oben beschriebene Substanzen oder durch entsprechende Maßnahmen zur lokalen Hemmung der Bildung oder Wirkung von Östrogenen, den Gehalt der Haut an Kollagenfasern zu vermehren.

Die topische Hemmung der Aromatas in der Haut kann dazu verwendet werden, lokal den Prozentsatz der Haut an Kollagenfasern anzuheben. Dies ist erfindungsgemäß bei der kosmetischen Behandlung von Falten im Gesicht und im Dekolleté oder bei der kosmetischen Beeinflussung von Schwangerschaftsstreifen oder Dehnungsstreifen an Unterbauch, Oberschenkeln und Gesäß (besonders gut sichtbar bei dunkler Hautfarbe) von Bedeutung.

Ein Teil der Substanzen und deren Fähigkeit zur Behandlung von gestörtem Unterhaut-Bindefettgewebe wie der Cellulite bzw. deren Einsatz zur Straffung und/oder Verkleinerung von Fettzellen-haltigen Körperpartien sind bereits aus der WO-A 97/36570 bzw. der WO-A 99/17712 bekannt. Jedoch spielen sich die dort beschriebenen Phänomene in dem Unterhaut-Bindefettgewebe der Subkutis ab, während dieser Hautaspekt der vorliegenden Erfindung auf den besonders kollagenhaltigen Teil der Haut, nämlich der Kutis (Epidermis und Korium) sowie anderer stark Kollagen-haltiger Teile des Körpers abzielt. Insbesondere finden sich in diesen Druckschriften keine Hinweise auf den überraschend gefundenen Zusammenhang zwischen einer Hemmung einerseits der Bildung und andererseits der Wirkung des lokalen Östrogens und einem positiven, direkten Einfluss auf das Kollagen lokal in der Kutis, die sowohl anatomisch wie funktionell von der Subkutis verschieden ist.

Folglich ist gemäß der vorliegenden Erfindung eine vollständige Penetrierung der Haut funktionell bis in die Subkutis nicht erforderlich, um die beschriebenen Wirkungen in der Kutis selbst zu entfalten. Die erfindungsgemäße positive Beeinflussung des Kollagens erfolgt über den relativen Östrogenmangel direkt in der Epidermis und im Korium der Kutis. Denn die Kutis der Haut ist in der Lage, aus Androgenen Östrogene zu bilden (Bulun 1998), da sowohl Fibroblasten (Macdiarmid 1994, Toda 1994, Staib 1994, Jakob 1995, Isurugi 1996) als auch Keratinozyten (Hughes 1997) über das Enzym Aromatase verfügen. Zudem sind Epidermis und Korium Östrogen-abhängige Hautschichten, müssen also über Östrogenrezeptoren verfügen (Hughes 1997 (Karatinozyten), Dieudonne 1998 (Fibroblasten)). Da die Aromataseaktivitat der Haut (besonders der weiblichen) konstitutiv ausgeprägt ist und Östrogene den Gehalt an Kollagenfasern vermindern, enthält die weibliche Haut konstitutiv nicht so viel Kollagenfasern wie die männliche Haut, die auch wesentlich dicker ist. Senkt man die lokale Östrogenkonzentration in der weiblichen Haut, kommt es zu einer Zunahme der Kollagenfasern. Da die Östrogenkonzentration der weiblichen Haut zu einem beträchtlichen Teil durch die lokale Aktivität der Aromatase bewirkt wird, wird eine Senkung der lokalen Östrogenkonzentration durch eine Hemmung der Aromataseaktivität in den Keratinozyten und den Hautfibroblasten erreicht. Die Hemmung der Haut-Aromatase führt also zu einer Zunahme der Kollagenfasern, speziell solche vom Typ I, und damit zur Zunahme von Dicke und Festigkeit der Haut. Diese Phänomene werden von den im Rahmen der Erfindung untersuchten Probandinnen nach etwa 4 Wochen Anwendungsdauer bemerkt und wurden experimentell nachgewiesen.

Die topische Hemmung der Aromatase in der Haut bringt es mit sich, dass dem Androstendion nur mehr der Weg zum Testosteron offen steht. Testosteron selbst kann auch nicht zu Östradiol aromatisiert werden, sondern wird bei aktiver 5-alpha-Reduktase in das starke Androgen DHT umgewandelt. Die Haut ist reich an 5-alpha-Reduktase (Mestayer 1996, Courchay, Luu-The 1994) und kann hierdurch das typisch männliche Aussehen vermitteln (Bart, Talgdrüsen, Dicke). Östradiol ist in der Lage, die 5 alpha Reduktase zu hemmen (Cassidenti 1991). Wird nun die Aromatase gehemmt, kommt es zur Verarmung der Haut an Östradiol. Die Hemmung und der Östradiolmangel sind zwei Faktoren, die der 5 alpha Reduktase ein erhebliches Übergewicht verleihen. Daher kann es sein, dass eine topische Hemmung nur der Aromatase zu einer gewissen Virilisierung der Haut führt. Hinzu kommt, dass Testosteron in geringen Mengen (6 Nanomol/L) die Aromatase in der Haut bereits halbmaximal hemmt (Berkovitz 1990). Durch Hemmung der 5 alpha Reduktase erreicht man eine Anreicherung der Haut mit Testosteron und damit auch eine Hemmung der Aromatase.

Besonders im Gesicht möchte man eine Virilisierung vermeiden. Daher ist es erfindungsgemäß vorgesehen, wenn zusammen mit der Aromatase stets auch die 5-alpha-Reduktase gehemmt wird, wenn man lokal den Kollagengehalt der Haut erhöhen möchte. Eine ausbleibende Hemmung der 5-alpha-Reduktase würde wohl den Kollagengehalt der Haut noch weiter zu steigern vermögen, dies aber auf Kosten der Virilisierung. Da es bei Hemmung beider Enzyme zur lokalen Kollagenvermehrung der Haut kommt, reicht hierzu anscheinend der alleinige Östrogenentzug aus. Eine begleitende Hemmung der Aromatase und der 5 alpha-Reduktase kann mit Sojasterole auch eine hemmende Wirkung auf die 5-alpha-Reduktase aus.

Für die positive Beeinflussung des Kollagens in der Kutis der Haut kann zur topischen Applikation der beschriebenen Wirksubstanz(en) bzw. der Zusammensetzung eine hierfür geeignete Formulierung des zu verwendenden Stoffs gewählt werden, z.B. eine Salbe, eine Creme, ein Gel, eine Emulsion (Lotio), ein Puder, ein Öl usw.. Zu diesem Zweck umfasst die Zusammensetzung Zusatzstoffe, die für die entsprechende Formulierung als Salbe, Creme, Gel, Emulsion, Puder oder Öl usw. üblich sind. Beschriebene sowie handelsübliche, herkömmliche Hautpflegemittel sind in den jeweiligen Formulierungen zum Einsatz in der vorliegenden Erfindung bestens geeignet. Als übliche Zusatzstoffe für solche Formulierungen dienen beispielsweise pflanzliche Öle wie Mandelöl, Olivenöl, Pfirsichkernöl, Erdnussöl, Rizinusöl u. dergl., Pflanzenextrakte, etherische Öle, Vitaminöle, Fette und fettähnliche Stoffe, Lipoide, Phosphatide, Kohlenwasserstoffe wie Paraffine, Vaseline, Lanolin, Wachse u. dergl., Detergentien, weitere Hautwirkstoffe wie Lecithin, Wollfettalkohole, Carotin u. dergl., Hautnährstoffe, Parfums, kosmetische Stoffe, Alkohole, Glycerol, Glykole, Harnstoff, Talk, Konservierungsmittel, Sonnenschutzmittel, Farbstoffe wie Titanweiß und Zinkweiß, Antioxidantien usw.. Als Grundsubstanz dient im allgemeinen Wasser, so dass - üblicherweise unter Zusatz von Emulgatoren wie Fettalkoholsulfate, Alkaliseifen, Lecitine, Triethanolamin u. dergl. - eine O/W- oder W/O-Emulsion erhalten wird.

### Andere Kollagen-haltige Körperregionen (nicht erfindungsgemäß)

Aufgrund der überraschend gefundenen Möglichkeit der Stabilisierung, Vermehrung und/oder Restaurierung des Kollagens in der Kutis der Haut mittels der speziellen Substanzen ist die Erfindung nicht nur dort, sondern auch auf andere, besonders Kollagen-haltige Bestandteile des Körpers als lokale Zielorte der Applikation anwendbar, wie Knorpel, Sehnen, Bänder, Fascien, Gefäßwände von Adern und Venen wie Krampfadern oder die Harnröhre, kollagenhaltige Geschwüre wie der Ulcus cruris, Zahnbein, Knochen, die Kollagenkapseln atherosklerotischer Plaques und dergleichen.

Eine typische das Bindegewebe betreffende Verletzung sportlicher Frauen ist die Ruptur des vorderen Kreuzbandes im Knie. Dies kommt bei Frauen wesentlich häufiger vor als bei Männern (Powell 2000, de Loes 2000). Die trifft vor allem auf den Zeitpunkt der Ovulation zu (Wojtys 2000). Dann finden sich auch die höchsten LH Spiegel im Blut und damit die stärkste extragonadale Bildung von Sexualsteroiden bei der prämenopausalen Frau. Anscheinend macht eine etwas höhere lokale Östrogenkonzentration innerhalb des Bandes dieses verletzlicher. Die Bänder des Kniegelenks verfügen über Östrogenrezeptoren (Sciore 1998).

So kann die Substanz bzw. die diese Substanz enthaltende Zusammensetzung ggf. auch unterstützend eingesetzt werden bei chirurgischen Eingriffen in solche, stark Kollagen-haltige Körperbestandteile. Bevorzugt ist der Einsatz für kosmetische Zwecke, auch im Rahmen der Schönheitschirurgie. Die erfindungsgemäß einzusetzende Substanz bzw. Zusammensetzung ist aber auch geeignet als Alternative zu chirurgischen Maßnahmen, beispielsweise um Knorpelmasse zu fördern oder aufzubauen oder um Sehnen und Bänder zu stärken. Ein weiteres vorteilhaftes Anwendungsgebiet ist daher auch nicht erfindungsgemäß die Sportmedizin, wo es gilt, den durch sportliche Beanspruchung verursachten Abnützungen oder gar Verletzungen von Sehnen, Bändern, Muskeln und Knorpeln entgegenzuwirken.

Für solche Applikationen eignen sich insbesondere topische Darreichungsformen wie jene, die oben im Zusammenhang mit Hautapplikationen beschrieben wurden, oder Injektionen von physiologisch verträglichen Lösungen oder Suspensionen, die eine oder mehrere der beschriebenen Substanzen enthalten, in oder an den gewünschten Zielort. Hinsichtlich der Injektionen richtet sich die Art der Trägerflüssigkeit und die übrigen Bestandteile nach dem jeweiligen Applikationsort und sind dem Fachmann geläufig.

### Osteoporose (nicht erfindungsgemäß)

Während der Menopause kommt es zu einem kontinuierlich schleichenden Verlust der Knochensubstanz. Da das Gerüsteiweiß des Knochens kontinuierlich abnimmt, kann dieser weniger Kalziumphosphat binden. Dies führt zu einer mit Röntgenmethoden feststellbaren Abnahme der Knochendichte. Dieser Vorgang konnte herkömmlich durch die Gabe von Östrogenen oder von Östrogenen und Gestagenen (hormonereplacement-therapy; HRT) verlangsamt werden. Auch die Gabe von Androgenen hat einen günstigen Effekt. Der Mechanismus der Knochenprotektion durch sehr geringe Konzentrationen an Östrogenen oder Androgenen im Blut, die dort zudem weitgehend an Transportproteine gebunden vorliegen, ist nicht bekannt.

Da Osteoblasten Aromatase enthalten (Shozu 1998, Tanaka 1996) ließen oben dargelegte Überlegungen vorhersagen, dass die Abnahme der Knochensubstanz auf dem kollagenvermindernden Effekt der gesteigerten lokalen Östrogenbildung beruht. Der Stimulus hierzu, die postmenopausal erhöhten LH-Konzentrationen, würde durch die Hormongaben deutlich vermindert, und damit würde die Knochenresorption aufgehalten - was den Effekt bei der klassischen HRT erklärt. Tatsächlich korreliert die Aromataseaktivität im Knochen mit dem Ausmaß der Osteoporose (Nawata 1995).

Dem neuen Konzept der Erfindung folgend ist es nunmehr möglich, durch eine Hemmung der Aromatase das Fortschreiten der Osteoporose aufzuhalten. Die Aromatase-Hemmung kann wahlweise topisch, durch Diffusion des steroidalen hydrophoben Arzneimittels durch die Haut, durch ein transdermales System oder durch einen systemisch in das Blut eingebrachten Hemmstoff, durch die Injektion eines Depots eines Arzneimittels in die Muskulatur oder durch Inhalation des Arzneimittels erfolgen. Für topische Applikationen eignet sich besonders das zugelassene 4-Hydroxy-Androstendion (Handelsname: Lentaron). Als dem Androstendion weitgehend identisches Sterol dringt es einfach durch die Haut und besetzt, wenn es auf die Aromatase trifft, irreversibel deren aktives Zentrum (Selbstmord-Hemmung; sucide inhibition). Eine orale Zufuhr ist somit obsolet.

Die Wirkung von Östrogenen kann auch durch die Blockierung des Östrogenrezeptors durch die oben genannten Anti-Östrogene erfolgen. Als die am verbreitetste Substanz bietet sich hier das Tamoxifen an, das bei der Hormontherapie des Brustkrebses angewandt wird.

### Übermäßige Glukokortikoidbildung im Körper und Vermeidung von Nebenwirkungen bei der Glukokortikoidtherapie (nicht erfindungsgemäß)

Wie in den Hautfibroblasten stimulieren Glukokortikoide auch die Aromataseexpression in den Osteoblasten (Shozu 1998, Tanaka 1996). Nach gängiger Lehrmeinung müsste dies eigentlich günstig für den Knochen sein. Die klinische Erfahrung zeigt aber eindeutig, dass die systemische Behandlung mit Glukokortikoiden zu einer vorzeitigen Osteoporose führt (Jardinet 2000, Lespessailles 2000). Dies lässt sich durch die oben angestellten Überlegungen einfach erklären, nicht hingegen durch die gängige Lehrmeinung.

Eine krankhafte, übermäßige Glukokortikoidbildung im Körper oder die Therapie mit Glukokortikoiden, wobei zu den Glukokortikoiden das Kortisol, das Kortison und das Kortikosteron zu zählen sind, wirkt sich auch schwächend auf andere Komponenten des Bindegewebes aus. Die Kollagenfasern in der Haut wurden im Zusammenhang mit den Dehnungsstreifen beim M. Cushing schon genannt. Auch der Zusammenhang mit der Stabilität von Sehnen und Bändern, die ja im wesentlichen aus Kollagenfasern bestehen, und Glukokortikoiden wird deutlich.

Unter der Therapie mit Glukokortikoiden reißen Sehnen häufiger (Leppilahti 1998). Da Glukokortikoide die Aromataseexpression stimulieren, ist es plausibel, dass die erhöhte Vulnerabilität von Sehnen wie der Achillessehne über diesen Mechanismus vermittelt wird. Glukokortikoide führen über eine Verminderung der Kollagensynthese in der Haut zu einer Atrophie derselben (Oikarinen 1998). Es ist plausibel, dass dies zumindest teilweise auf der Stimulierung der Aromataseexpression in Hautfibroblasten beruht, so dass die begleitende, wahlweise topische oder systemische Aromatasehemmung gemäß der Erfindung die nachteiligen Auswirkungen einer Therapie mit Glukokortikoiden, jedenfalls die auf Haut und Bindegewebe bezogenen Nebenwirkungen, zu vermeiden vermag.

Herzinfarkt, Hirninfarkt und atherosklerotische Plaques (nicht erfindungsgemäß)

Schon in der Kindheit kommt es zu Ablagerung von Lipiden in das Innere der Arterienwand. Diese weißlichen Lipide bilden zusammen mit den Makrophagen, die sie teilweise enthalten einen sogenannten "fatty streak". Er besteht überwiegend aus Cholesterinestern. Aus dem "fatty streak" kann sich mit der Zeit der atherosklerotische Plaque entwickeln (Schwartz 1993). Hierbei handelt es sich um eine größere Erhebung in der Intima, die von einer Kapsel aus Kollagenfasern umgeben ist und in ihrem inneren aus amorphen Cholesterinestern und lebenden oder zugrundegegangenen Makrophagen besteht (Libby 2000). Solange die Kollagenfaserkapsel intakt ist, macht sich der atherosklerotische Plaque klinisch nicht bemerkbar. Reißt diese aber ein, kommt das Blut mit dem Inhalt in Kontakt, der sehr schnell eine intravasale Blutstillung mit nachfolgendem Fibrinogenthrombus auslösen kann. Dieser Thrombus kann dann das arterielle Gefäß verstopfen und so die Sauerstoffzufuhr für nachgeschaltete Gewebsareale unterbinden und zu Herzinfarkt oder Hirninfarkt führen. Die Kapsel reißt umso leichter ein, je dünner sie ist, je weniger viskös der Inhalt des Plaques ist und je mehr Makrophagen der Plaque enthält, die kollagenabbauende Enzyme sezernieren können (Libby 1996, Davies 1994). Ein wichtiges therapeutisches Ziel im Rahmen der vorliegenden Erfindung ist die Plaque-Stabilisierung. Es kommt nicht so sehr darauf an, die Größe des Plaques zu vermindern, sondern ihn so zell- und lipidarm wir möglich zu bekommen und so zu stabilisieren.

Eine wesentliche Ursache für die drastische Zunahme der Herzinfarkte nach der Menopause könnte darin liegen, dass die Aromatase in der Gefäßwand, vor allem aber in den Makrophagen der Plaques, durch die permanent erhöhten LH-Konzentrationen stimuliert wird, und die gebildeten Östrogene den Kollagenstoffwechsel der Plaques so beeinflussen, dass er instabil wird. Tatsächlich enthalten die glatten Muskelzellen der Arterienwand eine ziemliche Menge an Aromatase (Harada 1999). Die Koronararterien können Östradiol synthetisieren und verfügen über Östrogenrezeptoren (Diano 1999). Eine Hemmung der Aromatase oder eine Blockierung von Östrogenrezeptoren hat somit nicht nur eine protektive Wirkung auf die Knochen, sondern auch auf die atherosklerotischen Plaques und kann so helfen, das Herzinfarktrisiko zu vermindern, insbesondere bei postmenopausalen Frauen.

### Harninkontinenz (nicht erfindungsgemäß)

Bei großen Hündinnen kommt es innerhalb von vier Wochen nach der Sterilisation häufig zur Harninkontinenz (Arnold 1997). Diese ist auch ein häufiges Problem älterer Frauen (Distler 2000). Der Effekt wird bei Hunden damit erklärt, dass die Schleimhaut der Harnröhre durch Sexualhormone ähnlichen zyklischen Schwankungen ihres Aufbaus unterliegt wie die Gebärmutterschleimhaut. Es ist anzunehmen, dass sich der Hund ein halbes Jahr im Anöstrus befindet, das heißt die Östrogenbildung in den Eierstöcken wird die meiste Zeit nicht stimuliert. Daher ist es fraglich, wie die Östrogene zur Harnkontinenz beitragen können. Östrogentherapie hat keinen deutlichen bessernden Effekt (Jackson 1999). Möglicherweise gibt es noch weitere Hormone aus dem Ovar und dem Hypothalamus bzw. Hypophyse, die sich wechselseitig beeinflussen und zum Teil die lokale Östrogensynthese stimulieren können.

Da die Dicke der Wand der Harnröhre und damit auch eventuell der Schließmechanismus von einem ausreichenden Anteil an Kollagenfasern abhängen dürfte, ist die Behandlung der Harninkontinenz bei älteren Frauen durch eine erfindungsgemäße Anwendung östrogenbildungs- oder wirkungshemmender Substanzen, etwa die systemische Therapie mit Aromatasehemmern, angezeigt.

Die systemische Therapie mit einem nebenwirkungsarmen Aromataseinhibitor hätte entgegen der Hormonersatztherapie den zusätzlichen Vorteil, dass das Gerinnungssystem nicht negativ beeinflusst wird und dass es nicht mehr zu Blutungen der Uterusschleimhaut kommt.

### Übermäßige Vitamin D - Produktion und "After Sun"-Anwendung (nicht erfindungsgemäß)

Neben Glukokortikoiden kann auch Vitamin D in Fibroblasten und Keratinozyten der Haut die Expression der Aromatase stimulieren (Hughes 1997). Vitamin D wird in der Haut durch Sonneneinstrahlung gebildet. Somit ist plausibel, dass Sonnenbaden den Kollagengehalt der Haut vermindert.

Daher besteht eine weitere nützliche Anwendung der Erfindung darin, zumindest die beschienenen Körperpartien mit einer topischen Anwendung der östrogenbildungs- oder -wirkungshemmenden Substanz zu behandeln, um so die beschleunigte Faltenbildung zu bremsen. Dies geschieht zweckmäßigerweise nach der am Tage erfolgenden Licht-/Sonneneinwirkung in einer "After Sun"-Anwendung, etwa in einer topischen Formulierung für die Nacht. Geeignet sind die bereits bei der Hautanwendung beschriebenen topischen Formulierungen, wobei wiederum wegen der guten Hautresorptionsfähigkeit die Aromatase-Inhibitoren und dort insbesondere die Sojasterole und deren Analoga bevorzugt sind. Zur Entgegenwirkung von virilisierenden Effekten ist wiederum eine gleichzeitige Hemmung von sowohl der Aromatase als auch der 5 alpha Reduktase vorteilhaft.

### Beeinflussung des Haarwachstums bei Männern und Frauen

Da die 5-alpha-Reduktase in der Haut vollständig hemmbar ist, und zwar beide Isoenzyme, ist es möglich, hierüber und unter gleichzeitiger Hemmung der Aromatase einen Einfluss auf die Geschwindigkeit des Wachstums der Körperbehaarung bei Männern und bei Frauen auszuüben. Die 5 alpha Reduktase ist auch für die Glatzenbildung beim Manne verantwortlich. Dies weiß man sicher daher, weil es gelingt, mit der systemischen Gabe von Finasterid den Haarwuchs auf der Kopfhaut zu stimulieren bzw. den Haarausfall zu bremsen (Whiting 1999, Brenner 1999). Minoxidil erhöht in der Kopfhaut die Aktivität der 17-beta HSD, wodurch Testosteron beschleunigt in Androstendion zurückverwandelt wird. Gleichzeitig wird aber auch die 5 alpha Reduktase stimuliert (Sato 1999). Finasterid und das Antiandrogen Flutamid bieten sich für eine topische Applikation auf den Haare verlierenden Kopf an. Dies wurde bisher an einem auf eine Nacktmaus transplantiertes Kopfhautmodell (Sintov 2000) und am Flankenorgan des Goldhamsters, um dort die Talgproduktion zu stoppen (Chen 1995), erfolgreich unternommen. Auch andere 5 alpha Reduktase -Hemmer wirkten dort gut topisch (Chen 1998). Topische Applikationen von Finasterid und einem Hemmer des Typ 1 Enzyms wurden auch an einem anderen Talgdrüsenmodell, der männlichen fuzzy Ratte getestet. Finasterid wirkte etwas besser als MK 386. Beide wirkten wesentlich schlechter als ein Antiandrogenrezeptorblocker (RU58841) (Ye 1997). Eine Substanz, die die 5 alpha Reduktase hemmt und zugleich als Antiandrogen wirkt (4-MA) wurde Affen über 27 Wochen auf den Kopf geschmiert. Die behandelten Affen wiesen im Gegensatz zu ihren unbehandelten Artgenossen keinerlei Anzeichen einer Glatze auf (Rittmaster 1987).

Dass man bisher nicht an eine topische Anwendung eines 5-alpha-Reduktaseinhibitors bei zuviel Körperbehaarung gedacht hat, geht daraus hervor, dass man das Mittel gegen Prostatahypertrophie Finasterid systemisch geben muss, um an der Kopfhaut die erwünschten Wirkungen zu erreichen. Finasterid wird auch zur Behandlung des Hirsutismus systemisch gegeben (Muderis 2000).

Im Rahmen der Erfindung wurde festgestellt, dass das Haarwachstum in Gesicht und Beinen bei Frauen nach Epilierung durch die gleichzeitige Hemmung von Aromatase und 5-alpha-Reduktase erheblich verlangsamt werden kann. Eine Kombination ist auch im Hinblick darauf vorteilhaft, dass im Falle der Hemmung nur der 5 alpha-Reduktase in der Haut der Extremitäten die Östrogenbildung in der Haut zunehmen würde. Verstärkend käme hinzu, dass Testosteron schneller verschwindet (wird zu Dihydrotestosteron), das ja die Aromatase hemmen kann. Die so gesteigerte Östrogenbildung in der Haut würde diese dünner machen und gleichzeitig das subkutane Fettgewebe vermehren. Deshalb ist es sinnvoll gleichzeitig die Aromatase zu hemmen.

Je nachdem, welcher Typ der 5-alpha Reduktase zusätzlich zur Aromatase gehemmt wird, lassen sich gewünschte Haarwachstumseffekte erzielen. Beispielsweise kann eine zusätzliche Hemmung beider Typen der 5 alpha Reduktase, etwa mit einem dem Androstendion sehr ähnlichen Sterol (4-Hydroxyandrostendion), möglicherweise sogar eine Wachstumshinderung nicht nur des Bartwuchses, sondern auch der Scham- und Achselhaare erzielt werden.

### Beispiele (nicht erfindungsgemäß)

Die Erfindung wird nachfolgend anhand einiger Beispiele erläutert.

### Beispiel 1) Augenfältchensalbe (25 ml)

| | |
|---|---|
| Cetylsterylalkohol | 3,5 ml |
| Natriumlaurylsulfat | 0,75 ml |
| Dünnflüssiges Paraffin | 5,0 ml |
| Weißes Vaselin | 15,5 ml |
| Oxidierte Sojaglycine mit | |
| Aromatasehemmwirkung | 0,15 ml |

1.1. 60 jähriger Mann mit stark ausgeprägter Fältchenbildung im Bereich der Augen, vor allem an Unter- und Oberlid:
   Nach 10-wöchiger 1 x täglicher Behandlung Straffung im Bereich der Augenlider, annähenderes Verschwinden der Fältchen.

1.2. 50 jährige Frau mit starker Fältchenbildung um die Augen, Zustand nach Lifting vor 5 Jahren.
   2 x tägliche Applikation von Beispiel 1):
      bereits nach 8-wöchiger Behandlung starke Straffung der Oberhaut im Bereich der Fältchenbildung um die Augen; nach 16 Wochen sind die Fältchen soweit verschwunden, dass die Frau angibt, dass sie nun besser aussehe als nach der Liftingprozedur.

### Beispiel 2) Gesichtscreme (50 ml)

| | |
|---|---|
| Propylenglykol | 12,5 ml |
| Isopropylmyristat | 3,0 ml |
| Sorbitanmonostorat | 0,5 ml |
| Polysorbat 80 | 1,0 ml |
| Stearylalkohol | 1,0 ml |
| Cetylsteorylalkohol | 3,0 ml |
| Glycerolmonostearat | 0,5 ml |
| Oxidierte Sojaglycine mit | |
| Aromatasehemmwirkung | 0,25 ml |
| Aqua dest. | ad 50,0 ml |

47-jährige Frau, starke Faltenbildung im unteren Gesichtsbereich, vor allem im Bereich von Wangen und Kinn:
Nach 6-wöchiger 2 x täglicher Behandlung mit Beispiel 2 war eine Straffung der Faltenbildung spür- und sichtbar; nach 12 Wochen waren nur noch leichte Fältchen sichtbar.

### Beispiel 3) Oberarm-Cremelotion (100 ml)

| | |
|---|---|
| Span 80 | 1,0 ml |
| Span 60 | 5,0 ml |
| Tween 60 | 9,0 ml |
| Propylenglykol | 15,0 ml |
| Palmitinsäure | 9,0 ml |
| Oxidierte Sojaglycine mit | |
| Aromatasehemmwirkung | 0,4 ml |
| Aqua dest. | ad 100,0 ml |

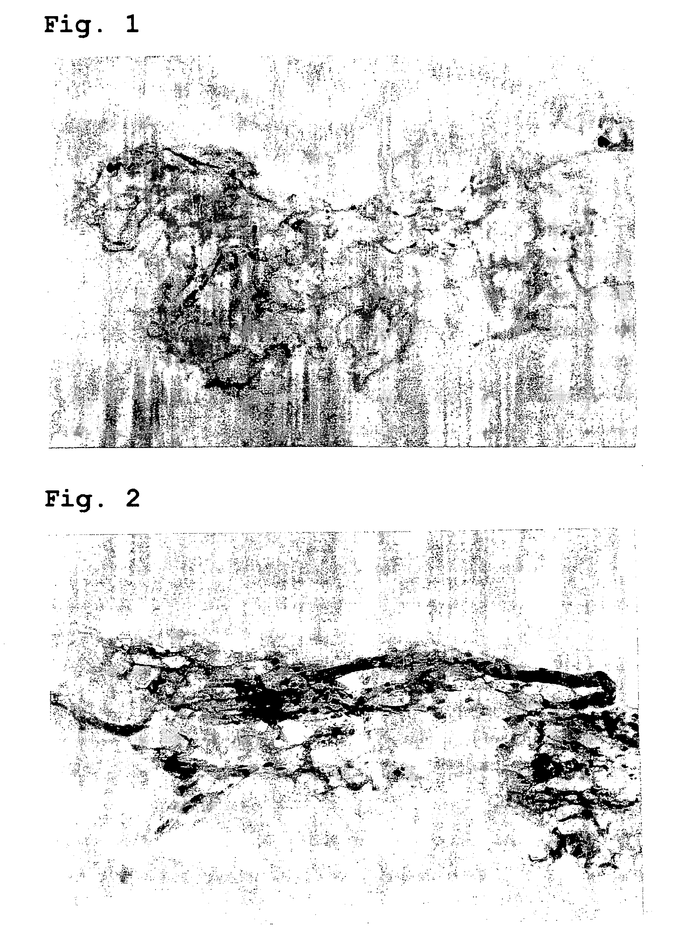

43-jährige Frau mit Oberhautfalten an den Oberarmen; 2 x tägliche Behandlung mit der Cremelotion: nach 4 Wochen deutliche Straffung spürbar; nach 8 Wochen deutliche Straffung spür- und sichtbar; nach 12 Wochen Faltenbildung stark reduziert; nach 16 Wochen Faltenbildung praktisch nicht mehr sichtbar. (Biospien Vor- und Nachbehandlung zeigen im mikrospopischen Bild die Zunahme kollagener Fasern in der Haut; Abb.1 vor Behandlung, Abb. 2 nach 16-wöchiger Behandlung)

### Beispiel 4) Gel gegen Dehnungs- bzw. Schwangerschaftsstreifen (100 ml)

| | |
|---|---|
| Mikrokristalline Cellulase | 4,0 ml |
| Polyethylenglykol 400 | 5,0 ml |
| Cetylakohol | 10,0 ml |
| Oxidierte Sojaglycine mit | |
| Aromatasehemmwirkung | 0,4 ml |
| Aquadest | ad 100,0 ml |

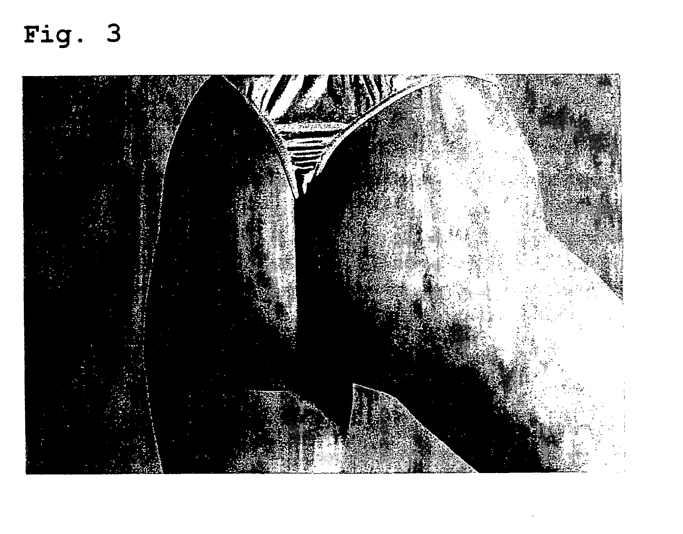

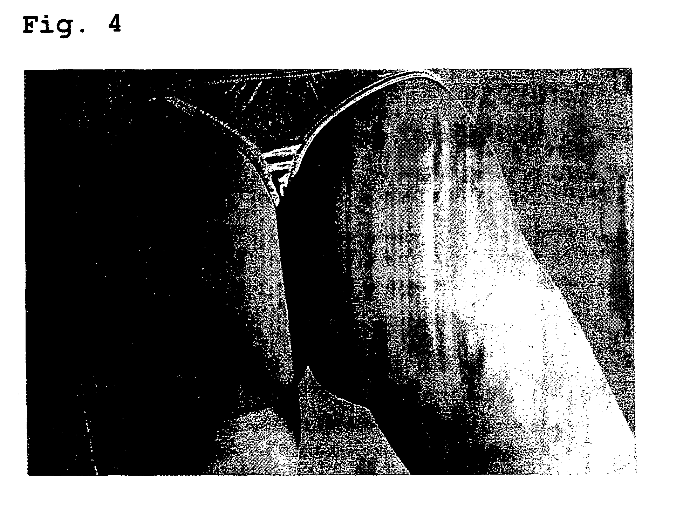

4.1. 27-jährige Frau, zweifache Mutter, starke Schwangerschaftsstreifen im Bereich des Abdomens; 2 x tägliche Anwendung der Gel-Creme Befunde: nach 6 Wochen leichte Straffung fühlbar; nach 12 Wochen Schwangerschaftsstreifen stark reduziert; nach 18 Wochen keine Schwangerschaftsstreifen mehr sichtbar.
4.2. 25-jährige Frau nach multiplen Diäten; Dehnungsstreifen in den Bereichen Oberschenkel und Gesäß. Befunde: nach 5 Wochen Rückgang der Dehnungsstreifen vor allem an den Oberschenkeln; bereits nach 10 Wochen Rückgang der Dehnungsstreifen an Gesäß und Oberschenkeln (fast) verschwunden; nach 15 Wochen alle Dehnungsstreifen praktisch nicht mehr sichtbar: Abb. 3 vor Behandlung, Abb. 4 nach 10-wöchiger Behandlung.

### Beispiel 5) Injektionsform zur intrafokalen Anwendung (1 ml)

15 mg 4-Hydroxi-Androstendion (Formestan) Hilfsstoffe: Benzylalkohol, Carmellose-Nd, Polysorbat, Natriumchlorid, Wasser 30-jährige Tennisspielerin (Bundesliga) mit starken Überdehnungserscheinungen in beiden Kniegelenken (Bänder und Sehnen), starke Schmerzen; Tennisspiel nur mit Bandagen beiderseits und unter Medikation mit nicht steroidalen anti-inflammatorischen Substanzen (NSAID) möglich. Injektion von Beispiel 5) in das Kniegelenk in 2-wöchigem Abstand über 10 Wochen: nach der 3. Injektion (4 Wochen) bereits deutlich verminderter NSAID-Verbrauch; nach 10 Wochen praktisch schmerzfreies Spiel (ohne NSAID!), Gefühl einer Stabilisierung der Kniegelenke.

### Literatur:

Arnold S, Schweiz Arch Tierheilkd 1997 : 139(6):271-6 Benson JR et al., Br J Cancer 1996 Aug; 74(3):352-8
Berkovitz GD et al., J Clin Endocrinol Metab 1984 Oct : 59(4) : 665-71
Berkovitz GD et al., J Clin Endocrinol Metab 1988 May : 66(5):1029-36
Berkovitz GD et al., Mol Cell Endocrinol 1990 Mar 5;69(2-3):187-97
Berkovitz GD et al., J Clin Endocrinol Metab 1992 Mar : 74(3):629-34
Bisat T et al., In Vitro Cell Dev Biol 1989 Sep : 25(9):806-12
Borel JP et al., Pathol Biol (Paris) 1984 Sep : 32(7):795-812
Brenner S and Matz H, Int J Dermatol 1999 Dec : 38(12):928-30
Brodie AM et al., Cancer Res 1989 Dec 1; 49(23) : 6551-5;
Published erratum appears in Cancer Res 1990 Jan 15 : 50(2):449
Cassidenti DL et al., Obstet Gynecol 1991 Jul; 78(1) :103-7 Chen C et al., J Invest Dermatol 1998 Aug; 111(2) : 273
Chen W et al., Dermatology 1996;193(3):177-84
Chen C et al., J Invest Dermatol 1995 Nov : 105(5):678-82
Courchay G et al., Skin Pharmacol 1996 : 9(3):169-76
Cummings SR et al., JAMA 1999 Jun 16 : 281(23):2189-97
Davies MJ et al., Basic Res Cardiol 1994 : 89 Suppl 1:33-9
de Loes M et al., Scand J Med Sci Sports 2000 Apr : 10(2):90-7
Diano S et al., Menopause 1999 Spring : 6(1):21-8
Dijkstra AC et al., J Invest Dermatol 1987 Jul : 89(1):87-92
Distler W, Z Arztl Fortbild Qualitatssich 2000 Apr : 94(3) : 211-5
Fujimoto M et al., J Clin Endocrinol Metab 1986 Aug : 63(2) :468-79
Gottlieb B et al., Am J Med Genet 1999 Dec 29 : 89(4):210-7
Gips H et al., J Endocrinol Invest 1980 Jan-Mar : 3(1):51-
Grino PB et al., Endocrinology 1990 Feb : 126(2):1165-72
Hamada K et al., J Invest Dermatol 1996 May : 106(5):1017-22
Harada N, Biochem Biophys Res Commun 1992 Dec 15 : 189(2):1001-7
Harada N et al., Circ Res 1999 Jun 11 : 84(11):1285-91
Hsiang YH et al., J Steroid Biochem 1987 Jan : 26; (1) : 131-5
Hughes SV et al., Endocrinology 1997 Sep : 138(9):3711-8
Iida S et al., J Clin Endocrinol Metab 1991 Jul : 73(1):192-6
Itami S et al., J Dermatol Sci 1994 Jul;7 Suppl: S98-103
Jackson S et al., Br J Obstet Gynaecol 1999 Jul : 106(7):711-8
Jardinet D et al., Rheumatology (Oxford) 2000 Apr : 39(4) : 389-92
Kwan G et al., Kidney Int 1996 Oct : 50(4):1173-9
Labrie F et al., Ann Endocrinol (Paris) 1995 : 56(1):23-9
Labrie F, Mol Cell Endocrinol 1991 Jul : 78(3) : C113-8
Labrie F et al., J Clin Endocrinol Metab 1997 Aug: 82(8):2403-9
Lachgar S et al., J Investig Dermatol Symp Proc 1999 Dec : 4(3):290-5
Lee KS et al., Clin Exp Dermatol 1994 Jul : 19(4):285-8
Leppilahti J and Orava S, Sports Med 1998 Feb : 25(2):79-100
Lespessailles E et al., Joint Bone Spine 2000 : 67(2):119-26
Libby P et al., Curr Opin Lipidol 1996 Oct : 7(5):330-5
Libby P, J Intern Med 2000 Mar : 297(3) : 349-58
Luu-The V et al., Invest Dermatol 1994 Feb : 102(2):221-6
Mestayer C et al., J Clin Endocrinol Metab 1996 May : 81(5):1989-93
Marttunen MB et al., Calcif Tissue Int 1999 Nov : 65(5):365-8
Milewich L et al., Placenta 1990 Mar-Apr : 11(2) : 95-108
Milewich L et al., Ann N Y Acad Sci 1988 : 548:66-89
Muderris II et al., Fertil Steril 2000 May : 73(5):984-7
Nawata H et al., J Steroid Biochem Mol Biol 1995 Jun : 53(1-6):165-74
Neugarten J et al., Am J Physiol 1999 Dec : 277(6 Pt 2):F875-81
Oikarinen A et al., Br J Dermatol 1998 Dec : 139(6):1106-10
Powell JW and Barber-Foss KD, Am J Sports Med 2000 May-Jun : 28(3):385-91
Rittmaster RS et al., J Clin Endocrinol Metab 1987 Jul; 65(1) : 188-93
Roberts AJ et al., Crit Rev Eukaryot Gene Expr 1995: 5(2):157-76
Sasano H et al., Endocr J 1999 Apr;46(2):233-42
Sato T et al., J Dermatol Sci 1999 Feb; 19(2) : 123-5
Schwartz CJ, Am J Cardiol 1993 Feb 25 : 71(6):9B-14B
Sciore P et al., J Orthop Res 1998 Sep : 16(5):604-10
Setnikar I et al., Arzneimittelforschung 1999 Aug : 49(8):708-15
Shozu M and Simpson ER, Mol Cell Endocrinol 1998 Apr 30 : 139(1-2) : 117-29
Silbiger S et al., Kidney Int 1999 Apr : 55(4):1268-76
Sintov A et al., Int J Pharm 2000 Jan 20 : 199(1) : 125-39
Stanczyk FZ et al., J Steroid Biochem Mol Biol 1990 Sep; 37(1) : 129-32
Svenstrup B et al., J Steroid Biochem 1990 May: 35(6):679-87 Tamaoka Y, Nippon Sanka Fujinka Gakkai Zasshi July 1987: 39(7):1129-36
Tanaka S et al., Endocrinology May 1996: 137(5):1860-9
Thiboutot D et al., J Invest Dermatol Sep 1998: 111(3):390-5
Theintz GE et al. Horm Res 1989 : 32(4):124-9
Whiting DA et al., J Investig Dermatol Symp Proc 1999 Dec : 4(3):282-4
Wojtys EM et al., Am J Sports Med 1998 Sep-Oct : 26(5):614-9
Venencie PY et al. , Br J Dermatol 1999 Sep : 141(3):438-46
Winkler EM et al., Am J Phys Anthropol 1993 Oct : 92(2):155-64
Ye F et al., Skin Pharmacol 1997;10(5-6):288-97
You S et al., Biol Reprod 2000 Jan : 62(1):108-16;
Zhao Y et al., Mol Endocrinol 1995 Mar : 9(3):340-9;

## Patentansprüche

1. Verwendung eines steroidalen Aromatase-Inhibitors, wobei der Aromatase-Inhibitor auch eine 5-alpha-Reduktase-Inhibitorwirkung besitzt, zur kosmetischen Behandlung von Falten im Gesicht und im Dekollete oder bei der kosmetischen Beeinflussung von Schwangerschaftsstreifen oder Dehnungsstreifen an Unterbauch, Oberschenkeln und Gesäß, wobei Kollagen stabilisiert, vermehrt und/oder restauriert wird.

2. Verwendung nach einem Anspruch 1, wobei es sich bei dem steroidalen Aromatase-Inhibitor um 4-Hydroxyandrostendion oder einem Derivat davon handelt.

3. Verwendung nach einem der vorangehenden Ansprüche, wobei es sich bei dem steroidalen Aromatase-Inhibitor um 4-Hydroxyandrost-4-en-3,17-dion handelt.

4. Verwendung nach einem der vorangehenden Ansprüche, wobei der steroidale Aromatase-Inhibitor in einer Zusammensetzung in einer Menge von 0,001 bis 5 Gew.-%, bezogen auf die Gesamtmenge der Zusammensetzung, enthalten ist.

5. Verwendung nach Anspruch 4, **dadurch gekennzeichnet, dass** die Zusammensetzung zur lokalen Applikation geeignet ist, vorzugsweise zur lokalen topischen Applikation.

6. Verwendung nach einem der vorangehenden Ansprüche, zur topischen Anwendung wobei gleichzeitig das Wachstum der Körperbehaarung verlangsamt wird.

## Claims

1. Use of a steroidal aromatase inhibitor, whereby the aromatase inhibitor also possesses a 5-alpha-reductase inhibitor effect, for cosmetic treatment of wrinkles in the face and cleavage or in cosmetic management of stretch marks due to pregnancy or stretch marks on the lower abdomen, thighs, and buttocks, whereby collagen is stabilized, increased and/or restored.

2. Use according to claim 1, whereby the steroidal aromatase inhibitor is 4-hydroxyandrostenedione or a derivative thereof.

3. Use according to any one of the preceding claims, whereby the steroidal aromatase inhibitor is 4-hydroxyandrost-4-ene-3,17-dione.

4. Use according to any one of the preceding claims, whereby the steroidal aromatase inhibitor is contained in a composition at a concentration of 0.001 to 5 wt.-%, with respect to the total amount of the composition.

5. Use according to claim 4, **characterized in that** the composition is suitable for local application, preferably for local topical application.

6. Use according to any one of the preceding claims for topical application, whereby the growth of body hair is slowed down simultaneously.

## Revendications

1. Utilisation d'un inhibiteur d'aromatase stéroïdien, dans laquelle l'inhibiteur d'aromatase possède également une action inhibitrice de 5-alpha-réductase en vue du traitement cosmétique de rides du visage et du décolleté ou lors de l'influence cosmétique de vergetures liées à la grossesse ou de vergetures sur le bas ventre, les cuisses et les fesses, dans laquelle du collagène est stabilisé, multiplié et/ou restauré.

2. Utilisation selon la revendication 1, dans laquelle il s'agit pour l'inhibiteur d'aromatase stéroïdien de la 4-hydroxyandrostèndione ou d'un dérivé de celle-ci.

3. Utilisation selon l'une quelconque des revendications précédentes, dans laquelle il s'agit pour l'inhibiteur d'aromatase stéroïdien de la 4-hydroxyandrost-4-èn-3,17-dione.

4. Utilisation selon l'une quelconque des revendications précédentes, dans laquelle l'inhibiteur d'aromatase stéroïdien est contenu dans une composition en une quantité de 0,001 à 5 % en poids par rapport à la quantité totale de la composition.

5. Utilisation selon la revendication 4, **caractérisée en ce que** la composition convient à l'application locale, de préférence à l'application topique locale.

6. Utilisation selon l'une quelconque des revendications précédentes pour l'application topique, dans laquelle la croissance de la pilosité corporelle est en même temps ralentie.
